# EUROPEAN PATENT APPLICATION

(11) **EP 0 813 847 A1**
(43) Date of publication of application: **29.12.1997**
(21) Application number: 97304106.4
(22) Date of filing: 12.06.1997
(51) Int. Cl.: A61B 19/02, A47B 23/00

(54) **Barrel top tray**

(30) Priority: 17.06.1996 US 664712
(71) Applicant: ELI LILLY AND COMPANY, Indianapolis, Indiana 46285 (US)
(72) Inventor: Lawton, Billy Carl, Indianapolis, Indiana 46208 (US); Simpson, Michael Don, Canyon, Texas 79105 (US); Young, Christopher Andrew, Hereford, Texas 79045 (US)
(74) Representative: Hudson, Christopher Mark

(57) **Abstract**

A barrel top tray includes a cylindrically-shaped body and means for attaching the body onto the top of a cylindrical support such as a barrel. The tray includes an upper surface which defines a central, flat work area and several recesses. The recesses are specifically configured to receive various items including bottles, instruments such as syringes, and a sponge.

## Description

### BACKGROUND OF THE INVENTION

### Field of the Invention:

The present invention relates to the field of work trays or tables for use in the preparation, handling and administration of animal medicines and the like. In particular, the invention relates to a tray which is adapted for mounting upon a barrel or similar structure.

### Description of the Prior Art:

In many settings it is desirable to have a work surface to facilitate the preparation and administration of various materials. One such need arises, for example, in connection with the handling of animal medicines.

In the past, it has been common to simply employ a make-shift arrangement of structures which happen to be already available. Many cattle processing areas have simply used the top of a barrel for this purpose. Of course, such surfaces are not especially adapted for containing the materials and equipment to be used, and there is the risk that items will spill or fall off of the barrel top. Such surfaces also may lack the desired level of cleanliness or orderliness.

There has remained a need for a method and device for containing equipment and materials in a clean, safe and useful manner. The present invention satisfies this need.

### SUMMARY OF THE INVENTION

Briefly describing one aspect of the present invention, there is provided a barrel top tray including a body portion defining a central work area and several recessed compartments and means for attaching the body to an external support. Compartments of different shapes and sizes are provided to accommodate reception of a variety of items, including bottles, instruments and cleaning materials.

It is an object of the present invention to provide a tray which is useful for supporting a variety of materials and implements, and which also provides a clean, convenient working surface.

A further object of the present invention is to provide a work tray which is adapted for mounting to existing support structures; such as barrels.

Further objects and advantages of the present invention will be apparent from the description of the preferred embodiment which follows.

### BRIEF DESCRIPTION OF THE DRAWINGS

Figure 1 is a perspective view of a tray constructed in accordance with the present invention.

FIG. 2 is a top, plan view of the tray of Figure 1.

FIG. 3 is a front, elevational view of the tray of Figure 1.

FIG. 4 is a left side, elevational view of the tray shown mounted upon a barrel.

FIG. 5 is a bottom, plan view of a preferred embodiment of the tray.

### DESCRIPTION OF THE PREFERRED EMBODIMENT

For the purposes of promoting an understanding of the principles of the invention, reference will now be made to the embodiment illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

The present invention provides a work tray which is readily mounted to a support. The tray has a variety of uses. For purposes of explanation, the tray is described herein with respect to its use in connection with the handling and administration of medications or the like to animals. The tray is attached to an external support, e.g. a barrel, and provides a work surface and associated compartments for receiving a variety of materials and instruments.

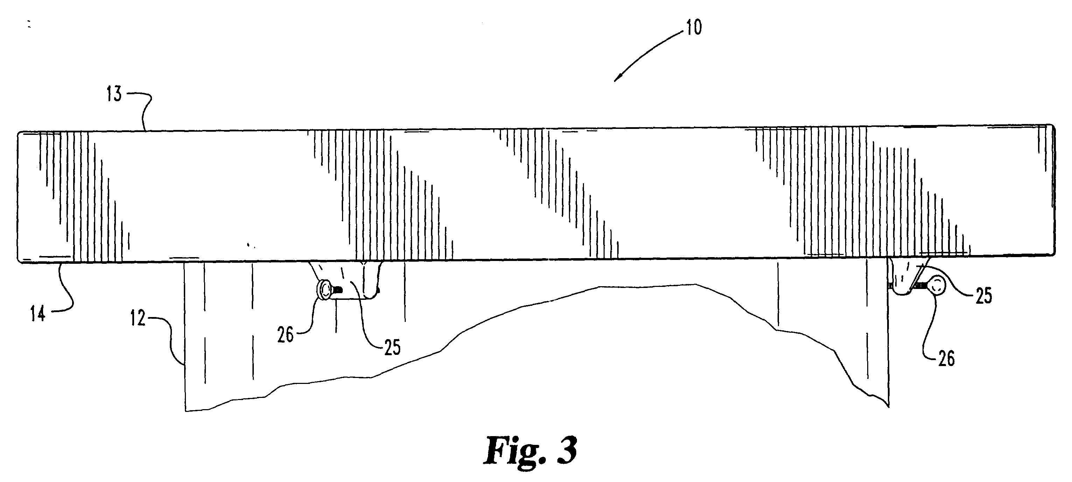

Referring in particular to the drawings, there is shown a barrel top tray 10 constructed in accordance with the present invention. The tray includes a body 11 sized to be received upon a cylindrical support 12, such as a barrel (Fig. 3). The body includes an upper surface 13 and a lower surface 14. The body may be formed from a variety of materials, and preferably comprises a molded plastic. As such, the interior is preferably hollow with internal support walls (not shown) to provide the desired rigidity while conserving weight and material.

The upper surface defines a central, flat work area 15 which is slightly recessed from the top of the tray. The recessing of the work area provides a surrounding rim which extends upwardly relative to the work area. The work area is useful for the handling of materials and instruments, while the surrounding rim retains fluids or the like which may spill in the area, as well as preventing instruments from falling off the work area. In use, the work area may also be utilized to contain promotional or informational material, either directly applied to the surface or attached to the surface by adhesives. Such informational material may include instructions for the use of particular items for which the tray may be particularly intended. In one embodiment, the tray is distributed for use with animal medicines, and the work area is provided with information concerning the preparation and administration of such medicines.

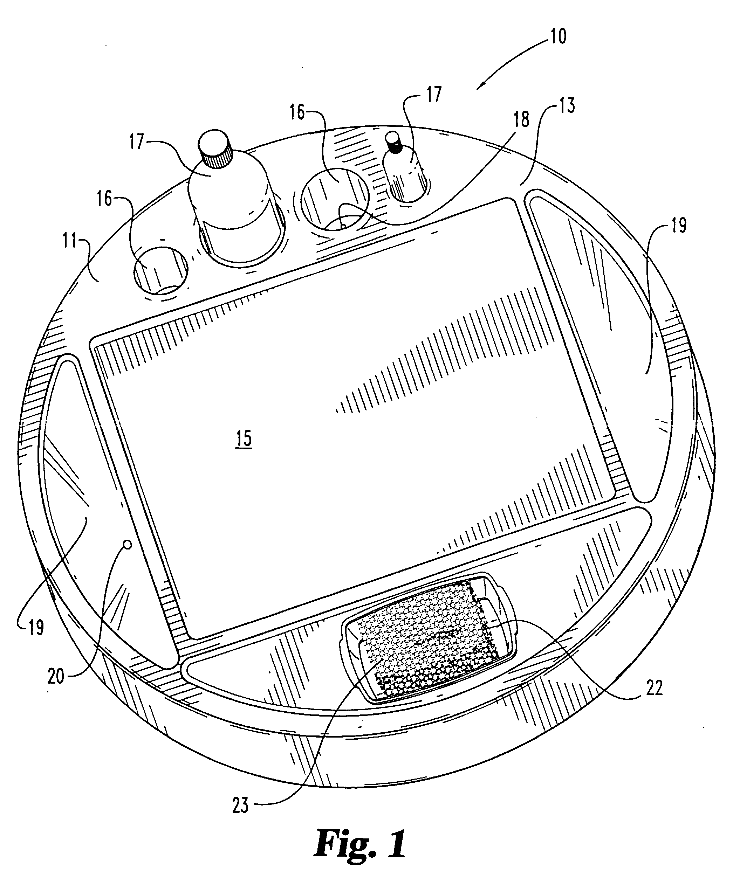

The upper surface of the body also defines several recessed compartments. One or more cylindrical recesses 16 are included for receiving cylindrically-shaped containers, such as bottles 17. In a preferred embodiment, the cylindrical recesses 16 are of different sizes, to accommodate different sized bottles. The several cylindrical recesses are preferably positioned adjacent one another along one side of the central work area, typically at the top or far end of the tray from the position of the user of the tray. If desired, drain holes 18 (Fig. 1) may be included at the bottoms of the cylindrical recesses.

The upper surface of the body also defines one or more elongated recesses 19 are included for receiving instruments or other devices. The bottoms of the elongated recesses are preferably slanted downwardly in the direction toward the interior of the tray to facilitate the reception of instruments therein. In a particular design, the tray includes two elongated recesses positioned on opposed sides of the tray, as shown in Fig. 1.

As indicated, a preferred embodiment of the tray is directed to use with the administration of animal medicines. In such instance, the elongated receptacles are useful for retaining syringes or the like, with one receptacle for new syringes and the other for used syringes. If desired, a drain hole, such as shown at 20 (Fig. 1) may be included at the bottoms of the elongated recesses, and the slanting of the bottoms of the recesses then facilitates the drainage of these compartments.

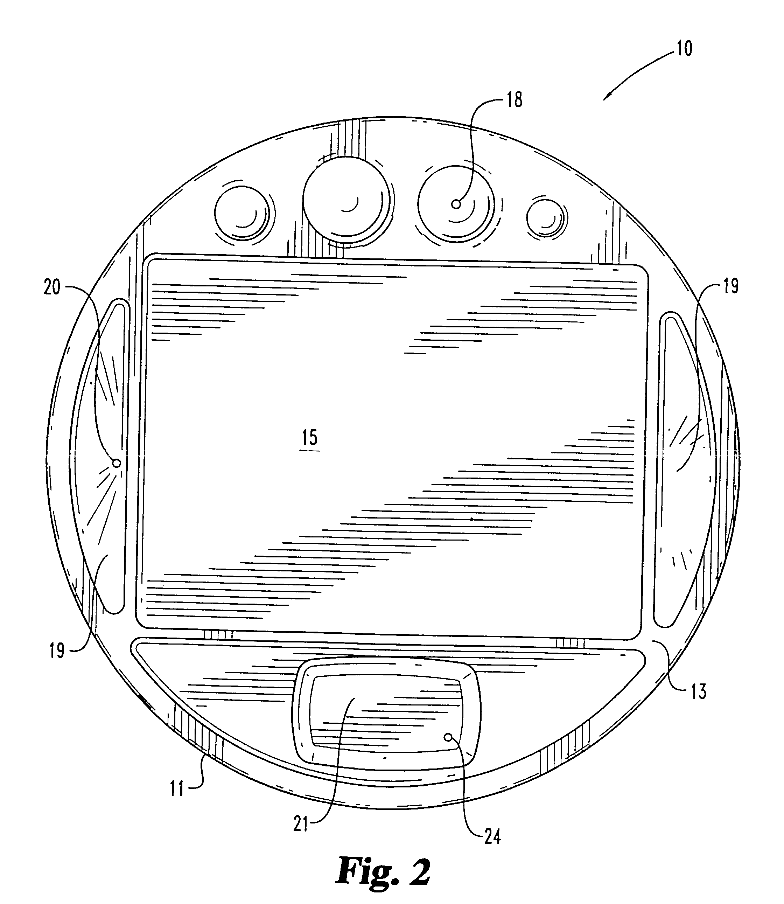

The tray also desirably includes a rectangular recess 21 (Fig. 2) useful for reception of a sponge or other cleaning material. The rectangular recess is preferably positioned between the pair of elongated recesses at the position opposite the cylindrical receptacles. This places the sponge or other cleaning material directly in front of the user of the tray during use. In Fig. 1 there is shown a plastic receptacle 22 and sponge 23 received within the recess 21. Optimally, the receptacle 22 may be provided with a cover (not shown) to be used when the tray is not in use. As for the other recesses, the rectangular compartment 21 may optionally be provided with a drain hole 24 (Fig. 2).

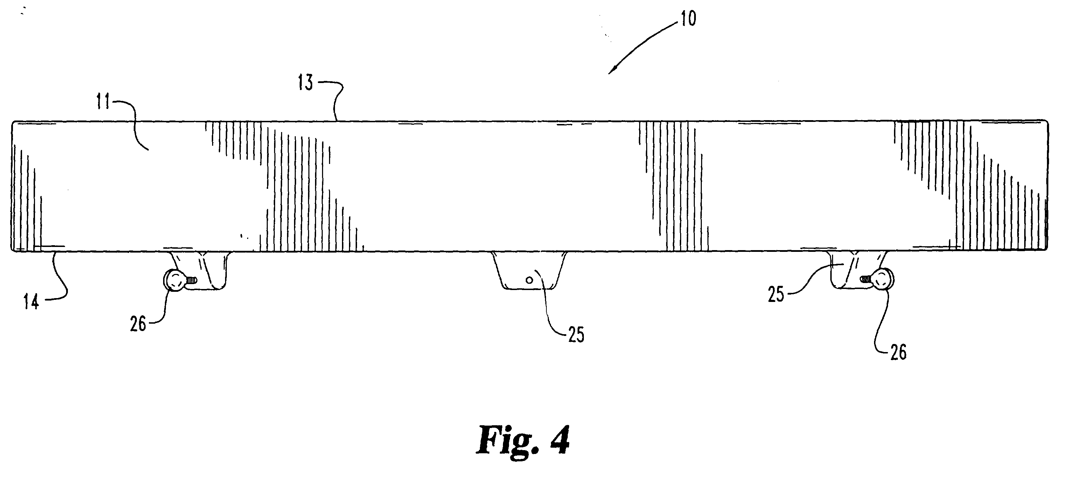

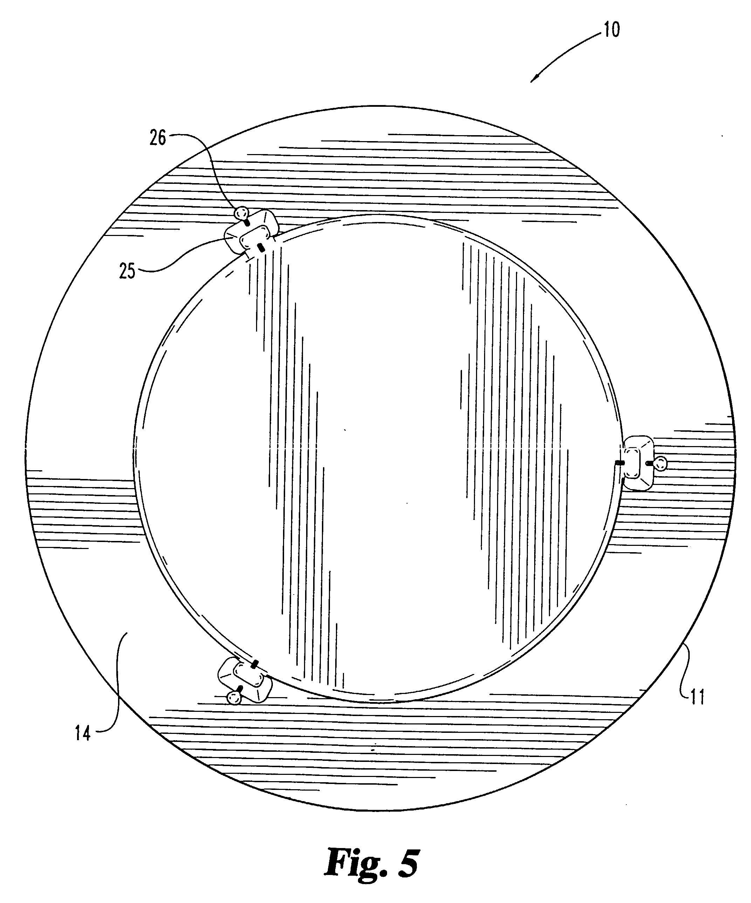

The tray is also provided with attachment means for attaching the tray to an external support. The attachment means are associated with the lower surface or underside of the tray body. As shown for example in Fig. 3-5, the tray is preferably provided with a plurality of legs 25 extending downwardly from the lower surface. Bolts 26 are threadingly received through the legs 25 and may be extended inwardly to engage the external support, such as barrel 12. Preferably at least three legs and bolts are employed to provide firm engagement of a cylindrical support. Naturally, the tray size and the position of the legs can be varied to accommodate attachment to different sized barrels or other cylindrical supports.

The barrel top tray of the present invention has a variety of uses. As previously noted, a preferred use is in connection with the preparation and administration of animal medicines. It is common for there to be 55 gallon steel or plastic barrels in an area where cattle or other animals may be handled. Many cattle processing areas already use such barrels as a work station, but the use of the barrel top alone is unsatisfactory by comparison to the present invention. In this type of setting, the tray of the present invention provides a simple and inexpensive device which affords a clean, convenient work surface with surrounding compartments for medicine bottles and instruments. A sponge soaked in an antiseptic solution is also included for use in cleaning needles or the like. The barrel top tray is applicable to all processing areas for cattle, including at dairies, stocker operations, feedyards, salebarns and veterinary clinics, and swine producers may similarly incorporate the tray in processing areas as well.

While the invention has been illustrated and described in detail in the drawings and foregoing description, the same is to be considered as illustrative and not restrictive in character, it being understood that only the preferred embodiment has been shown and described and that all changes and modifications that come within the spirit of the invention are desired to be protected.

## Claims

1. A barrel top tray comprising:
a cylindrical body sized to be received upon a cylindrical support, said body including upper and lower surfaces, the upper surface defining a central, flat work area and several recessed compartments; and
attachment means associated with the lower surface of said body for attaching said body to a support.

2. The tray of claim 1 in which the upper surface defines an upwardly-extending rim surrounding the central work area.

3. The tray of claim 1 in which the upper surface of said body defines a plurality of cylindrical recesses for receiving cylindrical containers therein.

4. The tray of claim 3 in which the cylindrical recesses are of at least two different sizes.

5. The tray of claim 3 in which the cylindrical recesses include drain holes at the bottoms thereof.

6. The tray of claim 1 in which the upper surface of said body defines a plurality of elongated recesses for receiving instruments therein.

7. The tray of claim 6 in which each of the elongated recesses includes a bottom which slants downwardly in the direction toward the interior of said body.

8. The tray of claim 6 in which there are a pair of elongated recesses positioned on opposed sides of said body exterior of the central work area.

9. The tray of claim 8 in which the elongated recesses include drain holes in the bottoms thereof.

10. The tray of claim 6 in which the upper surface of said body also defines a plurality of cylindrical recesses for receiving cylindrical containers therein.

11. The tray of claim 10 in which there are a pair of elongated recesses positioned on opposed sides of said body exterior of the central work area and in which the plurality of cylindrical recesses are positioned together between the pair of elongated recesses.

12. The tray of claim 1 in which the upper surface defines a rectangular recess for receiving a sponge unit therein.

13. The tray of claim 12 in which the rectangular recess includes a drain hole at the bottom thereof.

14. The tray of claim 12 and further including a sponge received within the rectangular recess.

15. The tray of claim 12 in which the upper surface of said body also defines a plurality of elongated recesses for receiving instruments therein.

16. The tray of claim 15 in which the upper surface of said body also defines a plurality of cylindrical recesses for receiving cylindrical containers therein.

17. The tray of claim 16 in which there are a pair of elongated recesses positioned on opposed sides of said body exterior of the central work area and in which the plurality of cylindrical recesses are positioned together between the pair of elongated recesses, the rectangular recess being positioned between the pair of elongated recesses exterior of the central work area and opposite the plurality of cylindrical recesses.
